# EUROPEAN PATENT APPLICATION

(11) **EP 4 327 656 A1**
(43) Date of publication of application: **28.02.2024**
(21) Application number: 21959905.7
(22) Date of filing: 06.10.2021
(51) Int. Cl.: A01N 1/02

(54) **CONTAINER FOR STORING BIOLOGICAL TISSUE**

(71) Applicant: Coretissue Bioengineering Inc., Yokohama-shi, Kanagawa, 230-0045 (JP)
(72) Inventor: SAKAI Shinichi, Yokohama-shi, Kanagawa 230-0045 (JP)
(74) Representative: Schön, Christoph
(86) International application number: PCT/JP2021/037015
(87) International publication number: WO 2023/058163

(57) **Abstract**

To provide a container for storing a biological tissue capable of storing the biological tissue with an immersed state suitable for use, that is, with its original form being maintained. A container for storing a biological tissue comprises an inner container used to store the biological tissue, and an outer container which seals and accommodates the inner container. The outer container comprises a gas permeation part which allows sterilization gas to flow into the inner container which is accommodated in the outer container from outside the outer container, or the outer container and the inner container are made of a material which allows permeation of a radioactive ray or an electron beam. The inner container comprises a container having an upper opening and a bottom and capable of accommodating the biological tissue and a solution which hydrates and moisturizes the biological tissue in a solid state, and a lid part which closes the opening. The outer container and the inner container are pierceable by an injection needle which injects the solution from outside the outer container.

## Description

### Technical field

The present invention relates to a container for storing a biological tissue, especially a biological tissue in a dry solid state, which is to be used after hydration and moisturization.

### Background Art

A tissue composed of a component of biological origin or the like (hereinafter referred to as a "biological tissue") is, for example, freeze-dried and then stored in a sterilized state. When in use, the tissue is re-hydrated before being used. Patent Literature 1 discloses a treatment method of such a biological tissue, for example, a biological tissue composed of a pericardium or a tendon, which method includes steps of drying and then sterilizing the tissue, wherein after the biological tissue is impregnated with oligosaccharide solution, the drying step of drying the biological tissue is performed, and then the sterilizing step of sterilizing the biological tissue with ethylene oxide gas at a temperature of about 30°C is performed.

### Citation List

### Patent Literature

Patent Literature 1: Japanese Patent No.6078838

### Summary of Invention

### Technical Problem

Biological tissues of this type are stored in a state of being sterilized by sterilizing gas, such as ethylene oxide gas, or by sterilizing means, such as transmissive electromagnetic wave or the like such as radioactive rays or electron beams. Accordingly, a container capable of transporting sterilized biological tissues is desired.

Among the biological tissues of this type, a freeze-dried biological tissue softens when hydrated. Accordingly, sheet-shaped biological tissues in particular may be damaged or be in a partially folded state when receiving external stimuli, impacts, or the like, during hydration. When hydrated in a partially folded state, the biological tissues may be in an unevenly immersed state, and the folded regions need to be expanded when in use.

In addition, in order to uniformly hydrate the biological tissues, it is necessary to immerse them in a solution evenly. However, since the specific gravity of biological tissues is substantially equal to water, the biological tissues float when hydrated, in some cases. When biological tissues are hydrated in a floating state, it causes a problem that the biological tissues are immersed unevenly.

The present invention has been made in view of the problem, and it is an object of the present invention to provide a container for storing a biological tissue, capable of transporting the biological tissue in a sterilized state.

It is another object of the present invention to provide a container for storing a biological tissue, capable of storing the biological tissue with its original form being maintained.

### Solution to Problem

In order to accomplish the obj ects, a container for storing a biological tissue of the present invention comprises an inner container used to store the biological tissue; and an outer container which seals and accommodates the inner container. The outer container comprises a gas permeation part which allows sterilization gas to flow into the inner container which is housed in the outer container from outside the outer container, or the outer container and the inner container are made of a material which allows permeation of a radioactive ray or an electron beam. The inner container comprises a container having an upper opening and a bottom and capable of accommodating the biological tissue and a solution which hydrates and moisturizes the biological tissue in a solid state, and a lid part which closes the opening. The outer container and the inner container are pierceable by an inj ection needle which injects the solution from outside the outer container.

According to such an aspect, when, for example, the container for storing a biological tissue is placed under reduced pressure and is allowed to stand still under the atmosphere of sterilization gas such as ethylene oxide gas, the sterilization gas flows into the outer container and into the inner container through the gas permeation part. As a result, the entire inner container and the biological tissue can be maintained in a sterilized state. Note that when the outer container and the inner container are made of a material which allows permeation of a radioactive ray or an electron beam, irradiating the outer container and the inner container with the radioactive ray or the electron beam makes it possible to maintain the entire inner container and the biological tissue in the sterilized state without providing the gas permeation part.

When, for example, a saline solution is used as the solution, the saline solution is encapsulated in a soft bag and is stored so as to be connectable to injection needles, such as syringe needles, through a tube to allow the saline solution to be used for drip infusion, and the like. Therefore, according to such an aspect, it is possible to inject a solution, such as a sterilized saline solution, into the inner container while maintaining an aseptic state by simply piercing the outer container and the inner container by an injection needle which injects the solution from outside the outer container. As a result, the injection of the solution into the inner container can be performed easily. Therefore, the biological tissue can be hydrated while the asepsis of the container for storing a biological tissue can be maintained. As a result, even when a biological tissue is hydrated in areas other than a clean field, it is possible to hydrate the biological tissue while maintaining the aseptic state. Once the biological tissue is sufficiently hydrated, the biological tissue can be transported to a clean field while the aseptic state is maintained.

In the present invention, it is preferable that the lid part or the container of the inner container comprises an injection port provided to allow injection of the solution into the container, and a mark is provided at a position of the outer container aligned with a position of the injection port to allow the injection needle, which injects the solution, to puncture and inject the solution into the inner container through the injection port.

According to such an aspect, it is possible to inject the solution through the injection port simply by allowing the injection needle to puncture the mark indicating the position to puncture.

In the present invention, it is preferable that a plug pierceable by the injection needle is provided at the position of the outer container aligned with the position of the injection port of the inner container.

According to such an aspect, it is possible to inject the solution into the inner container through the injection port simply by allowing the injection needle to puncture the plug. In addition, in the case where the plug is an elastic material, such as rubber, the injection needle, when it is inserted, is allowed to inject the solution while the sealed state being maintained. After the injection needle is removed, the place pierced by the injection needle is closed. Therefore, the asepsis can be maintained even after the injection needle is inserted and removed.

In the present invention, it is preferable that the inner container comprises a unit which retains the biological tissue in the container with an original form of the biological tissue being maintained, and the container or the lid part comprises the injection port of the solution at a position where the solution, injected into the inner container from outside the outer container, is allowed to immerse and hydrate the biological tissue retained in the container.

According to such an aspect, since the container or the lid part comprises the injection port for the solution at a position where the solution is allowed to immerse and hydrate the biological tissue retained in the inner container, it is possible to pour the solution so that the liquid surface is located above the position where the unit retains the biological tissue. Since the inner container comprises the unit which retains the biological tissue in the container with an original form of the biological tissue being maintained, it is possible to store the biological tissue while preventing the biological tissue from being unevenly immersed when the biological tissue is immersed with the solution.

In the present invention, it is preferable that the unit includes, in the inner container, a pair of gripping parts provided on the bottom part of the inner container and the lid part which closes the opening of the container, and the pair of gripping parts grips and retains the biological tissue from above and below.

According to such an aspect, since the pair of gripping parts grips and retains the biological tissue from above and below, it is possible to stably store the biological tissue. By removing the lid part from the inner container, the retaining of the biological tissue can be released, which makes it possible to easily take out the biological tissue.

In the present invention, it is preferable that a recessed part is provided in an area where the gripping parts face each other, the recessed part complying with a cross-sectional shape of the biological tissue in a dry and solid state, so that the gripping parts retain the biological tissue inserted into the recessed part.

According to such an aspect, since the biological tissue is inserted into the recessed part and retained, the biological tissue can be stored more stably.

In the present invention, it is preferable that the inner container is formed from a flexible material, and the lid part comprises a projection part configured to fit into the opening of the container when the lid part closes the opening, so that when side walls of the container are pressed and bent, the projection part slips upward on an inner surface of the container and detaches from the container.

When the biological tissue is immersed in the solution in the container having the lid part, the lid part is removed from the container and the biological tissue is take out after the elapse of a predetermined time from the start of the immersion. According to such an aspect, a medical professional can pop up and remove the lid part from the container by simply pressing the side walls of the container. Therefore, it is possible to improve the efficiency of the operation for removing the biological tissue from the container.

### Brief Description of Drawings

FIG. 1 is an exploded perspective view of a container for storing a biological tissue as an embodiment of the present invention.
FIG.2 is a top view of the container for storing a biological tissue as an embodiment of the present invention.
FIG.3 is an exploded perspective view of an inner container in FIG. 1.
FIG.4 is a sectional view of the inner container in FIG. 1.
FIG.5 is an explanatory view showing an aspect of injecting a solution into the inner container in FIG.1.
FIG. 6 is an explanatory view showing an aspect of introducing sterilization gas into the inner container of the container for storing a biological tissue.
FIG. 7 is an explanatory view showing an aspect of injecting the solution into the inner container of the container for storing a biological tissue.
FIG. 8 is a sectional view of a container for storing a biological tissue according to a modification 1.
FIG. 9 is a sectional view of an inner container of a container for storing a biological tissue according to a modification 2.
FIG. 10 is a sectional view of a container for storing a biological tissue according to a modification 3.

### Description of Embodiments

### Embodiment

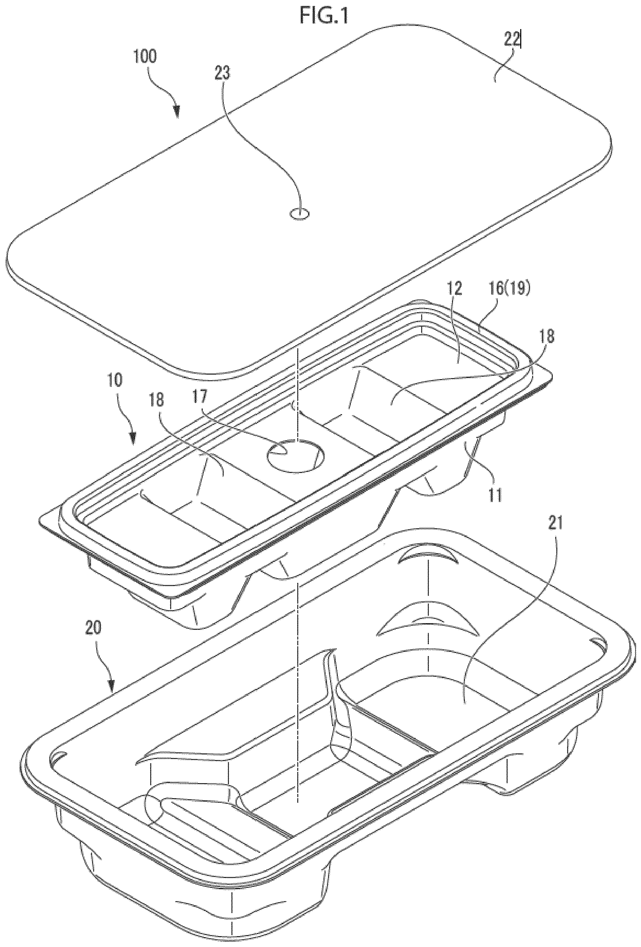

A container for storing a biological tissue 100 as an embodiment of the present invention as shown in FIG. 1 comprises an inner container 10, and an outer container 20 which seals and accommodates the inner container 10.

The inner container 10 comprises a container 11 having an upper opening and a bottom and capable of accommodating a solution and a biological tissue 30, and a lid part 12 which closes the opening. The inner container 10 is not particularly limited as long as it is resistant to (insoluble in) the solution, though the inner container 10 is preferably made of a flexible material which transmits light such as transparent or translucent material. Although polyethylene terephthalate (PET) is preferable as the flexible material, examples of the flexible material may include thermoplastic resin materials, such as low density polyethylene (LDPE), high density polyethylene (HDPE), polyvinyl chloride (PVC), polypropylene (PP), oriented polypropylene (OPP), ethylene vinyl acetate (EVA), polystyrene (PS), nylon (NYL), oriented nylon (ONY), polyvinylidene chloride (PVDC), ethylene vinyl alcohol copolymer (EVOH), and copolymers, mixtures, and laminates thereof. The thermoplastic materials may contain colorants, ultraviolet absorbers, light stabilizers, antistatic agents, and reinforcing materials such as glass fibers. The inner container 10 may be formed from a material pierceable by an injection needle which injects a solution.

The outer container 20 is formed to have an upper opening and a bottom and is also formed into a substantially rectangular parallelepiped shape capable of accommodating the inner container 10. The outer container 20 is not particularly limited as long as it is resistant to (insoluble in) the solution, though the outer container 20 is preferably made of a material which transmits light such as a transparent or translucent material. Although polyethylene terephthalate (PET) is preferable as the material, examples of the material may include thermoplastic resin materials, such as low density polyethylene (LDPE), high density polyethylene (HDPE), polyvinyl chloride (PVC), polypropylene (PP), oriented polypropylene (OPP), ethylene vinyl acetate (EVA), polystyrene (PS), nylon (NYL), oriented nylon (ONY), polyvinylidene chloride (PVDC), ethylene vinyl alcohol copolymer (EVOH), and copolymers, mixtures, and laminates thereof. The outer container 20 is formed from a material pierceable by an injection needle which injects the solution.

A bottom part 21 of the outer container 20 is formed to be fittable with a bottom part 13 of the inner container 10. In the present embodiment, the bottom part 13 of the inner container 10 is formed in a recessed shape in accordance with first protruding parts 14. The bottom part 21 of the outer container 20 is formed in a protruding shape so as to be fittable with the bottom part 13 of the inner container 10.

The outer container 20 has an air permeable sheet 22 as the gas permeation part which allows sterilization gas to flow in from the outer container 20. The air permeable sheet 22, in the present embodiment, is provided so as to close an upper opening of the outer container 20. Note that the gas permeation part may partially be provided.

The air permeable sheet 22 as the gas permeation part has air permeability which allows permeation of sterilization gas such as ethylene oxide. The air permeable sheet 22 is not particularly limited as long as it has the air permeability. Examples of the air permeable sheet 22 may include sterilization paper, and non-woven fabrics. Note that examples of the sterilization paper may include Tyvek (registered trademark) manufactured by DuPont.

In the center of the air permeable sheet 22, a plug 23 is provided as a mark of the position to puncture by an injection needle 40. Although the material of the plug 23 is not particularly limited, it is preferable to be an elastic material which can be punctured by the needle, for example. Although the elastic material is not particularly limited, examples of the elastic material may include various rubber materials, such as natural rubber, butyl rubber, isoprene rubber, butadiene rubber, styrene-butadiene rubber, silicone rubber, epichlorohydrin rubber, ethylene propylene rubber, nitrile rubber, and fluororubber, various thermoplastic elastomers, such as polyurethane-based, polyester-based, polyamide-based, olefin-based, and styrene-based elastomers, and copolymers, mixtures, laminates, and foams thereof. The elastic materials may contain vulcanizing agents, vulcanizing accelerators, colorants, ultraviolet absorbers, light stabilizer, antistatic agents, or the like. Note that the mark may be an indicator of the position to puncture by the injection needle 40 and that the plug 23 may not be provided. For example, the mark may be an area colored so as to be distinguishable from other areas.

The injection needle 40 is not particularly limited as long as a flow path is formed to allow the solution to flow. For example, infusion needles, suction needles, bottle needles, microcannula, and non-coring needles (Huber needles) may be used. It is preferable to use, out of these injection needles 40, a non-coring needle (Huber needle) from the viewpoint of preventing damage to the plug 23, and it is more preferable to use a resin bottle needle.

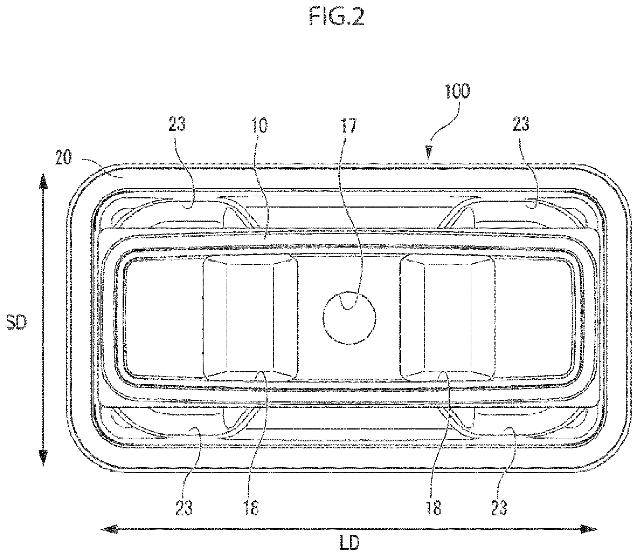

As shown in FIG. 2, in the present embodiment, the inner container 10 is formed in a rectangular shape in a top view. The outer container 20 can accommodate the inner container 10 so as to surround the periphery of the inner container 10 in a top view. Specifically, the inner container 10 is accommodated in such a way that the gap 33 are formed between an outer wall of the inner container 10 and an inner wall of the outer container 20 in its shorter-side direction SD.

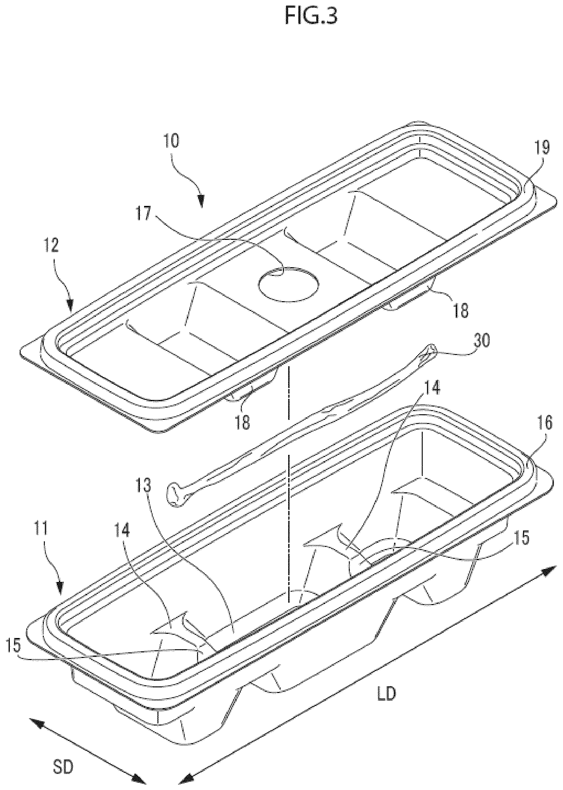

As shown in FIG. 3, the inner container 10 is a container usable for storing the biological tissue 30 used after hydrating and moisturizing a dry solid state.

As the biological tissue 30, tissues collected from animals such as pigs, cows, horses, or humans and subjected to drying treatment, such as freeze-drying or vacuum-drying, can be used. As the sites of such biological tissues, those with an extracellular matrix structure can be used. Examples of the sites may include liver, kidney, ureter, bladder, urethra, tongue, tonsil, esophagus, stomach, small intestine, large intestine, anus, pancreas, heart, blood vessel, spleen, lung, brain, bone, spinal cord, cartilage, testis, uterus, fallopian tube, ovary, placenta, cornea, skeletal muscle, tendon, nerve, skin, fascia, pericardium, dura mater, umbilical cord, heart valve, cornea, amnion, intestinal tract, small intestine submucosa, and other collagen-containing tissues.

For example, in the case of a fascia (pericardium), sheet-shaped biological tissues with a length and width of 10 cm x 10 cm and a thickness of about 1 to 2 mm are often used. In the case of the tendon, rod-shaped or string-shaped biological tissues with, for example, a diameter of 7.5 to 8 mm (dry state: 5 to 6 mm) and a length of about 16 cm are often used. In the present embodiment, an example of using a string-shaped artificial tendon as the biological tissue 30 will be described.

Although the solution used for hydrating the biological tissue 30 is not particularly limited, examples of the solution may include saline, distilled water, phosphate buffered saline (PBS), alcohol solutions such as ethanol, biological fluids such as serum, and mixtures thereof. Additives may be added to the solution to the extent that they do not interfere with the purpose of the present invention. Examples of such additives may include chemicals such as antibiotics and preservatives, cells or cell extracts, anti-inflammatory agents, proteoglycans, painkillers, hemostatic agents, epidermal growth factors, fibroblast growth factors, nerve growth factors, keratin cell growth factors, platelet-derived growth factors, vasoactive intestinal peptides, stem cell factors, bone morphogenetic proteins, and chondrocyte growth factors.

The container 11 is formed into a substantially rectangular parallelepiped shape. The container 11 has first protruding parts 14, which are formed so as to protrude from the bottom part 13 toward the upper opening. The first protruding parts 14 are provided in a pair so as to have an interval therebetween in a longer side-direction LD of the container 11.

An edge part is formed so as to surround the opening of the container 11. The edge part comprises a first projection part 16 formed so as to annularly project upward. The first projection part 16 is formed in a tapered shape with its width being gradually narrowed from its base part to top end.

The lid part 12 is formed into of a substantially rectangular plate shape. The lid part 12 has an injection port 17 formed so as to extend through its thickness direction, in its central part in the longer side-direction LD. In other words, the injection port 17 is provided at the position where the solution is allowed to immerse and hydrate the biological tissue 30 retained in the inner container 10. The injection port 17 can also introduce sterilization gas to a storage space enclosed by the inner wall surfaces and the bottom part of the inner container 10.

The lid part 12 has second protruding parts 18, which are formed so as to protrude toward the bottom part 13. The second protruding parts 18 are provided in a pair so as to have an interval therebetween in the longer side-direction LD of the lid part 12 and to have the injection port 17 interposed therebetween. The second protruding parts 18 are also formed at the positions facing the first protruding parts 14 in the longer side-direction LD of the inner container 10. The first protruding parts 14 have recessed parts 15 provided at areas facing the second protruding parts 18. In other words, the recessed parts 15 are provided in the areas where the pair of first protruding parts 14 and the pair of second protruding parts 18 face each other.

The lid part 12 has an edge part, in which a second projection part 19 is formed so as to annularly project upward. The second projection part 19 is formed into a hollow so as to be fittable with the first projection part 16.

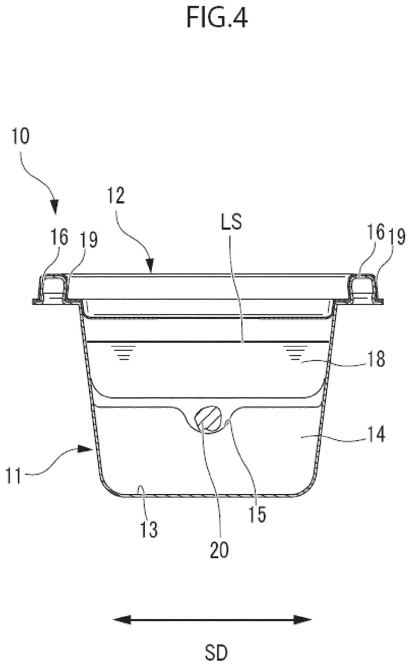

As shown in FIG. 4, the respective first protruding parts 14 are formed over inner wall surfaces facing each other in the shorter-side direction SD of the container 11. Thus, since the first protruding parts 14 are formed over the inner wall surfaces facing each other, the mechanical strength of the container 11 in the shorter-side direction SD can be enhanced.

In the middle of tip parts of the respective first protruding parts 14, the recessed parts 15 are formed so as to be recessed toward the bottom part 13. The recessed parts 15 are formed into the shape complying to the cross-sectional shape of the biological tissue 30. In the present embodiment, the biological tissue 30 is formed into a rod (cylindrical) shape, and so the recessed parts 15 are formed into the shape of a semicircle arc.

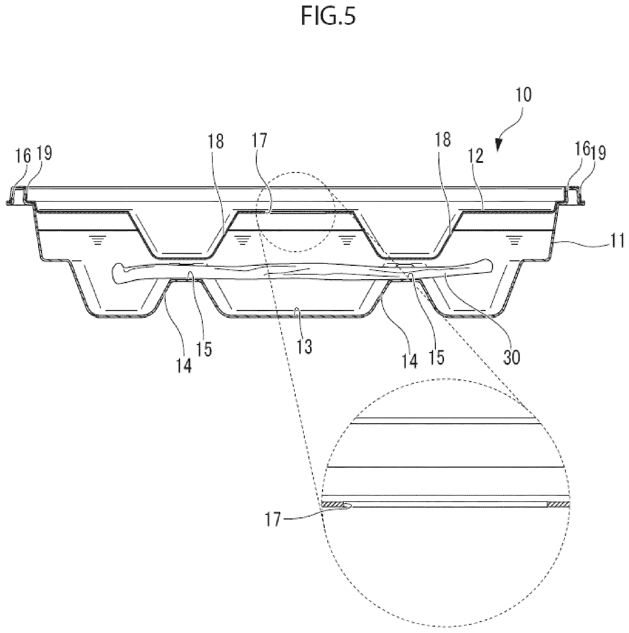

As shown in FIG. 5, the first protruding parts 14 and the second protruding parts 18 of the inner container 10 can grip the biological tissue 30 in an up-down direction of the inner container 10. That is, the first protruding parts 14 and the second protruding parts 18 function as a pair of gripping parts provided on the bottom part 13 of the inner container 10 and on the lid part 12. In other words, the first protruding parts 14 and the second protruding parts 18 function as a unit which grips the biological tissue 30 from above and below to retain the biological tissue 30 in the inner container 10 with an original form of the biological tissue 30 being maintained.

The injection port 17 is provided above the position where the unit retains the biological tissue 30. Therefore, it is possible to pour the solution so that a liquid surface LS of the solution is located above the position of retaining the biological tissue 30. Note that the inner container 10 may be made of a material which allows the position of the liquid surface LS of the solution in the inner container 10 to be visually recognized, for example, a material which transmits light such as a transparent or translucent material.

When the solution is injected into the inner container 10 so that the liquid surface LS of the solution is above the second protruding parts 18 (above the position of retaining the biological tissue 30), it becomes possible to secure the amount of the solution required for hydrating the biological tissue 30. When the liquid surface LS of the solution is above the position of retaining the biological tissue 30, the biological tissue 30 may float in the solution. When the biological tissue 30 is gripped with the pair of protruding parts in the up-down direction, the floating of the biological tissue 30 can be prevented. As a result, it becomes possible to retain the original form of the biological tissue 30 and to retain the biological tissue 30 in the state of being located in the solution, so that the biological tissue 30 can uniformly be immersed in the solution.

Since the biological tissue 30 retained by the unit can be released by removing the lid part 12, the medical professional can easily take out the biological tissue 30 from the inner container 10. When, for example, the inner container 10 is pressed and bent in the shorter-side direction SD (an arrow in the drawing) in such a manner that both the side walls are closer to each other at the time of removing the lid part 12, the lid part 12 can be removed with one hand.

Specifically, when both the side walls of the inner container 10 are bent, the lid part 12 slips upward like sliding over the inner surface of the second projection part 19 and detaches from the container 11, since the first projection part 16 is formed in a tapered shape.

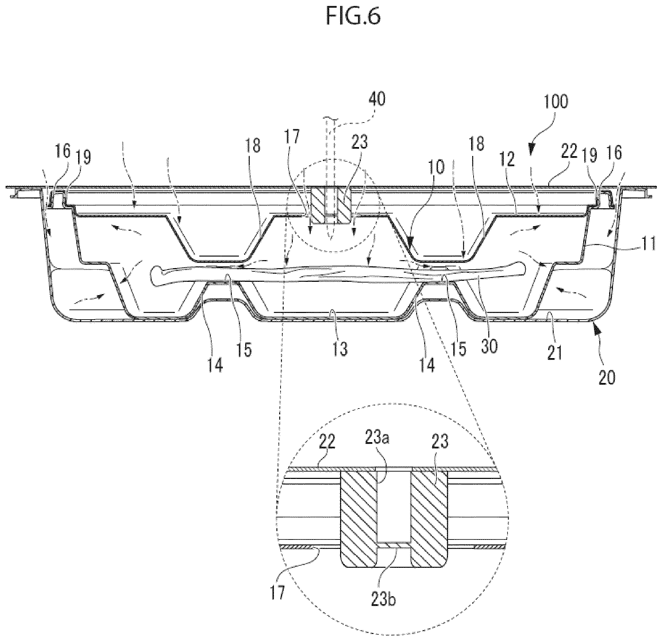

As shown in FIG. 6, the plug 23 provided in the air permeable sheet 22 is provided in alignment with the position of the injection port 17 of the lid part 12 in the inner container 10. The plug 23 has an insertion hole 23a formed in its center part so as to be recessed in a thickness direction. The insertion hole 23a may be formed to allow insertion of the injection needle 40 and to be equal in diameter to the injection needle 40. The insertion hole 23a has a closing part 23b which closes the insertion hole 23a on the top end side which is the side to be inserted into the injection port 17. Therefore, when the plug 23 is punctured by the injection needle 40, the injection needle 40 is retained in the insertion hole 23a. This makes it possible to inject the solution directly into the inner container 10 while retaining the injection needle 40 in the insertion hole 23a of the plug 23. In other words, the plug 23 constitutes at least part of a flow path of the solution flowing from the outer container 20 to the inner container 10.

Description is given of how to use the container for storing a biological tissue 100 which has been described in the foregoing.

A manufacturer fits the bottom part 13 of the inner container 10 with the bottom part 21 of the outer container 20 and accommodates the inner container 10 in the outer container 20.

Thus, when the outer container 20 and the inner container 10 are formed to be fittable with each other, unexpected movements of the inner container 10 can be prevented when the container for storing a biological tissue 100 is transported. As a result, it is possible to prevent unwanted shocks to the biological tissue 30.

The manufacturer puts the biological tissue 30, as if inserting the biological tissue 30, in the recessed parts 15 of the container 11 in the inner container 10 and closes the opening with the lid part 12, so that the pair of protruding parts 14 and 18 retains the biological tissue 30. Thus, when the biological tissue 30 is inserted into the recessed parts 15, it becomes possible to reliably retain the biological tissue 30.

After accommodating the inner container 10 in the outer container 20, the manufacturer closes the upper opening of the outer container 20 with the air permeable sheet 22. Note that the air permeable sheet 22 may be attached to the outer container 20 by, for example, thermal fusion.

The manufacturer allows the container for storing a biological tissue 100 to stand still under the atmosphere of sterilization gas, such as ethylene oxide gas, and performs sterilizing treatment. Specifically, the container for storing a biological tissue 100 is allowed to stand still in a chamber, and after the temperature in the chamber is adjusted to a predetermined temperature, vacuum-decompression is performed. When the inside of the chamber reaches a prescribed degree of decompression, water vapor and sterilization gas, such as ethylene oxide gas, are ejected to fumigate the inside of the chamber.

In FIG. 6, since the sterilization gas, such as ethylene oxide, indicated by an alternate long and short dash line, is ejected in a vacuum state, the sterilization gas diffuses in the chamber and is introduced into the outer container 20 through the air permeable sheet 22. The sterilization gas introduced into the outer container 20 travels downward and comes into contact with the inner wall surfaces, and the like, of the outer container 20, the surface of the lid part 12 of the inner container 10, and the outer wall surfaces, and the like, of the container 11.

Then, the sterilization gas travels into the inner container 10 through the injection port 17 of the lid part 12 in the inner container 10. The sterilization gas traveling into the inner container 10 travels downward and comes into contact with the inner wall surfaces of the inner container 10, and the like, and the surface of the biological tissue 30. Thus, the regions of the outer container 20 and inner container 10 which have come into contact with the sterilization gas are put in an aseptic state (clean state), and therefore the biological tissue 30 can be stored.

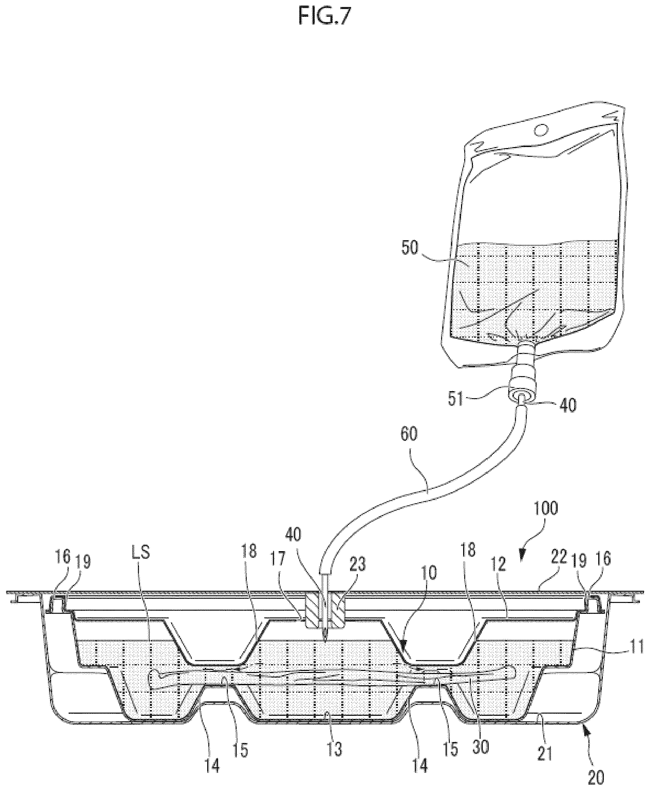

As shown in FIG. 7, when the medical professional hydrates the biological tissue 30, a saline solution contained in, for example, a bag-like container 50, such as a pouch, can be used as the solution. The container 50 comprises a connecting member 51 which can be punctured by the injection needle 40.

Therefore, when the connecting member 51 is punctured by one of the injection needles 40 provided at both the ends of a tube 60, and the plug 23 serving as a mark is punctured by the other injection needle 40 from outside the outer container 20, the solution flows from the container 50 and is guided to the inner container 10. The medical professional injects the solution into the inner container 10 so that the liquid surface LS of the solution is above the retention position of the biological tissue 30. When injection of the solution is finished, the medical professional removes the injection needle 40 from the plug 23 and allows the container for storing a biological tissue 100 to stand still to hydrate the biological tissue 30. Since the outer container 20 and the inner container 10 are made of a material which allows the position of the liquid surface LS of the solution in the inner container 10 to be visually recognized, for example, a material which transmits light such as a transparent or translucent material, the liquid level can be recognized.

When, for example, a saline solution is used as the solution, the saline solution is encapsulated in a soft bag and is stored so as to be connectable to injection needles, such as syringe needles, through a tube to allow the saline solution to be used for drip infusion, and the like. Therefore, it is possible to inject the solution, such as the saline solution, into the inner container 10 by simply puncturing the plug 23 by the injection needle. As a result, the injection of the solution into the inner container 10 can be performed easily. Therefore, it is possible to hydrate the biological tissue while maintaining the asepsis of the container for storing a biological tissue.

The time (immersion time) for immersing the biological tissue 30 in the solution is determined appropriately depending on the biological tissue 30. The immersion time of the biological tissue 30 is, for example, about 10 minutes in the case of a fascia, such as a pericardium, or the like, and 3 to 8 hours in the case of a tendon.

After the lapse of the immersion time, which is determined depending on the biological tissue 30, the medical professional visually confirms the state of hydration of the biological tissue 30 in the container for storing a biological tissue 100. When the state of hydration of the biological tissue 30 is not adequate, the medical professional continues the immersion until the hydration state becomes adequate. When the inner container 10 and the outer container 20 are made of a material which transmits light such as a transparent or translucent material, the medical professional can confirm the hydration state of the biological tissue 30 without performing an operation of opening the air permeable sheet 22 and the lid part 12.

When the hydration state of the biological tissue 30 is adequate, the medical professional takes out the inner container 10 from the outer container 20. For example, the medical professional can carry the container for storing a biological tissue 100 into a clean field after performing hydration in a non-clean field. In this case, since the gap 33 are formed between the outer wall of the inner container 10 and the inner wall of the outer container 20, the medical professional in the clean field can take out the inner container 10 by inserting his or her fingers into the gap 33. Therefore, the container for storing a biological tissue 100 can be carried into the clean field while the outer side of the inner container 10 can also be maintained in the sterilized state, and the inner container 10 can be disposed in the clean field in a clean state (aseptic state).

The medical professional removes the lid part 12 of the inner container 10 from the container 11 and takes out the biological tissue 30. When, for example, the inner container 10 is pressed and bent in the shorter-side direction SD in such a manner that both the side walls are closer to each other at the time of removing the lid part 12, the lid part 12 can be removed with one hand.

Specifically, when both the side walls of the inner container 10 are bent, the lid part 12 slips upward like sliding over the inner surface of the second projection part 19 and detaches from the container 11, since the first projection part 16 is formed in a tapered shape. Without being limited to such an aspect, the lid part 12 may be removed by the medical professional pinching a pair of hollows, which are provided on the upper surface of the lid part 12 in accordance with the second protruding parts 18, with fingers.

Thus, when the biological tissue 30 is input into the container 11 under the atmosphere of sterilization gas, and the solution is injected into the container 11, the biological tissue 30 can be hydrated while the entire inner container 10 is maintained in the sterilized state.

Although ethylene oxide is used as sterilization gas in the sterilizing treatment, the sterilization gas is not particularly limited as long as the sterilization gas has sterile properties. For example, ozone and hydrogen peroxide can be used. The sterilizing treatment is not limited to the treatment with sterilization gas. For example, the sterilizing treatment can be performed by configuring the outer container 20 and inner container 10 with a material which can transmit transmissive electromagnetic wave such as: radioactive rays such as gamma rays; electron beams; or X-rays and irradiating the outer container 20 and the inner container 10 with the transmissive electromagnetic wave. Thus, when the sterilizing treatment is performed without using the sterilization gas, the treatment can be performed without providing the gas permeation part.

### [Alternative Embodiment 1]

In the above embodiment, the injection port 17 is provided in the lid part 12. However, the lid part 12 may have the injection port 17 formed at the position where the solution injected into the inner container 10 is allowed to immerse and hydrate the biological tissue 30 retained in the inner container 10. Accordingly, the injection port 17 may be formed in an upper part of the side wall of the container 11.

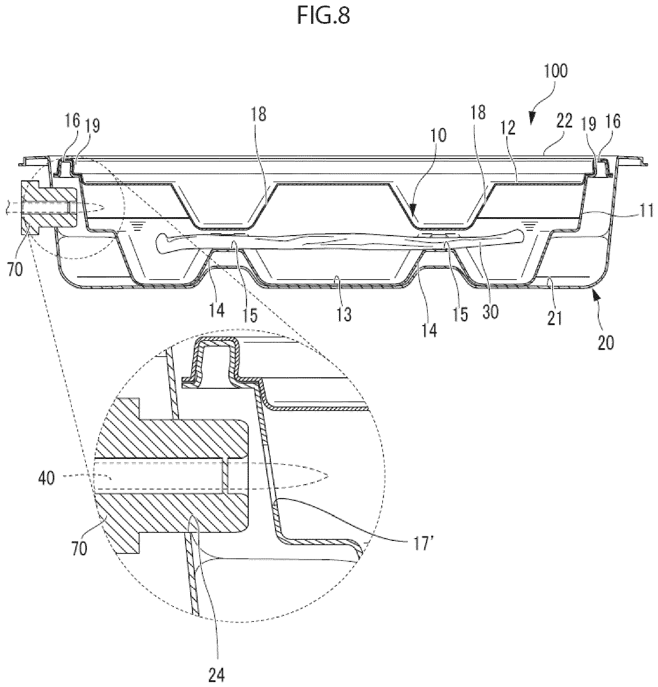

As shown in FIG. 8, the inner container 10 has an injection port 17' formed on the upper side of its side wall so as to extend through the side wall in the thickness direction. In addition, on the side wall of the outer container 20, there is an opening 24 formed at the position corresponding to the injection port 17' of the inner container 10 so as to extend through the side wall in the thickness direction. The opening 24 may be closed by a plug 70 which can be punctured by the injection needle 40.

According to such an aspect, the medical professional can puncture the connecting member 51 of the pouch-shaped container 50, which is filled with a solution, by one of the injection needles 40 provided at both the ends of the tube 60, and puncture the plug 70 with the other injection needle 40 to hydrate the biological tissue 30.

### [Alternative Embodiment 2]

The inner container 10 in the above embodiment forms a pair of gripping parts with the two first protruding parts 14 and the two second protruding parts 18 to grip the biological tissue 30. However, the aspect of retaining the biological tissue 30 is not limited thereto, and a pair of gripping parts may be formed with one first protruding part 14 and one second protruding part 18.

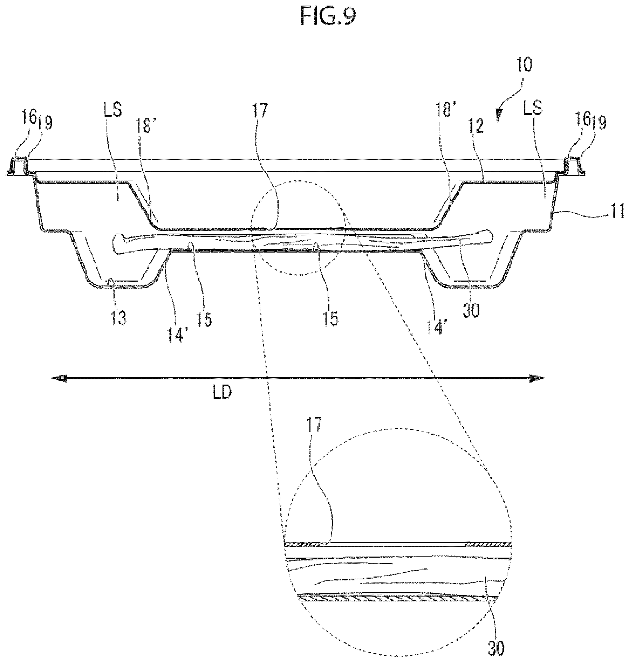

As shown in FIG. 9, a first protruding part 14' is a pedestal-shaped part extending upward from the bottom part 13 of the container 11 and extending along the longer side-direction LD. Note that a recessed part 15 is formed along the longer side-direction LD of the first protruding part 14'.

A second protruding part 18' is a pedestal-shaped part extending from the lid part 12 to the bottom part 13 and extending along the longer side-direction LD.

Thus, when the first protruding part 14' and the second protruding part 18' are provided, the biological tissue 30 is inserted into the recessed part 15 formed on the first protruding part 14', and the lid part 12 of the inner container 10 is closed, so that the biological tissue 30 is retained in the state of being placed between the first protruding part 14' and the second protruding part 18'.

Thus, when the unit which retains the biological tissue 30 is provided, the biological tissue 30 can be supported along the longer side-direction LD of the inner container 10, which makes it possible to prevent the biological tissue 30 from deforming during storage and hydration. Note that when the biological tissue 30 is retained in this way, it is possible to prevent deformation of the biological tissue 30 which is tubular and flexible, such as blood vessels, in particular.

### [Alternative Embodiment 3]

In the embodiment disclosed, the outer container 20 is formed so as to be able to fit with the inner container 10 and to accommodate the inner container 10. However, the outer container 20 is not limited to such an aspect as long as it can accommodate the inner container 10. For example, the outer container 20 may be formed into a bag shape.

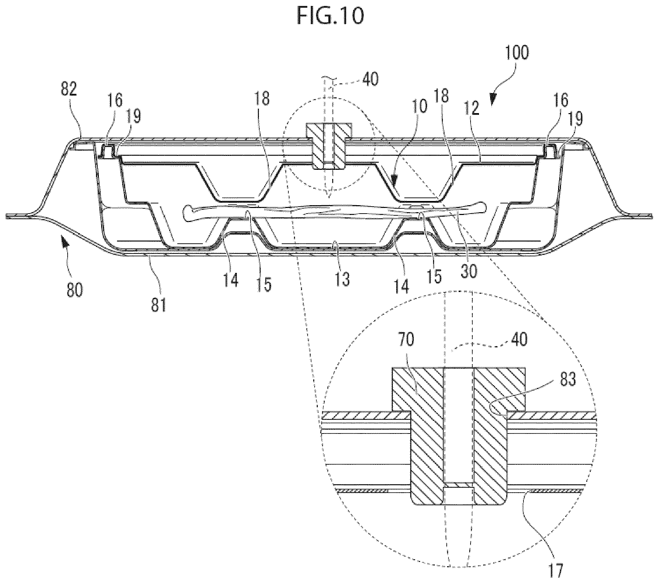

As shown in FIG. 10, an outer container 80 is formed into a bag shape by overlapping (by bonding perimeters of) an air permeable sheet 81 and a resin film 82 which is formed into a sheet shape. In substantially the center of the resin film 82, an opening 83 is formed in its thickness direction.

A manufacturer places the inner container 10 in the outer container 80 so that the position of the opening 83 is aligned with the position of the injection port 17. The manufacturer then closes the opening 83 with a plug 70 and inserts the plug 70 into the injection port 17. A medical professional can inject a solution into the inner container 10 by punctuating the plug 70 by the injection needle 40. Therefore, when the outer container 80 is configured in this way, it is possible to provide the container for storing a biological tissue 100 with a simple configuration.

### Reference Signs List

- 100: Container for storing a biological tissue
- 10: Inner container
- 11: Container
- 12: Lid part
- 14: First protruding part (gripping part)
- 17: Injection port
- 18: Second protruding part (gripping part)
- 20: Outer container
- 30: Biological tissue

## Claims

1. A container for storing a biological tissue, comprising:
an inner container used to store the biological tissue; and
an outer container which seals and accommodates the inner container, wherein
the outer container comprises a gas permeation part which allows sterilization gas to flow into the inner container which is housed in the outer container from outside the outer container, or the outer container and the inner container are made of a material which allows permeation of a radioactive ray or an electron beam,
the inner container comprises a container having an upper opening and a bottom and capable of accommodating the biological tissue and a solution which hydrates and moisturizes the biological tissue in a solid state, and a lid part which closes the opening, and
the outer container and the inner container are pierceable by an injection needle which injects the solution from outside the outer container.

2. The container for storing a biological tissue according to claim 1, wherein
the lid part or the container of the inner container comprises an injection port provided to allow injection of the solution into the container, and a mark is provided at a position of the outer container aligned with a position of the injection port to allow the injection needle, which injects the solution, to puncture and inject the solution into the inner container through the injection port.

3. The container for storing a biological tissue according to claim 2, wherein a plug pierceable by the injection needle is provided at the position of the outer container aligned with the position of the injection port of the inner container.

4. The container for storing a biological tissue according to claims 1 to 3, wherein
the inner container comprises a unit which retains the biological tissue in the container with an original form of the biological tissue being maintained, and the container or the lid part comprises the injection port of the solution at a position where the solution, injected into the inner container from outside the outer container, is allowed to immerse and hydrate the biological tissue retained in the container.

5. The container for storing a biological tissue according to claim 4, wherein
the unit includes, in the inner container, a pair of gripping parts provided on a bottom part of the inner container and the lid part which closes the opening of the container, and
the pair of gripping parts grips and retains the biological tissue from above and below.

6. The container for storing a biological tissue according to claim 5, wherein
a recessed part is provided in an area where the gripping parts face each other, the recessed part complying with a cross-sectional shape of the biological tissue in a dry and solid state, so that the gripping parts retain the biological tissue inserted into the recessed part.

7. The container for storing a biological tissue according to claims 1 to 6, wherein
the inner container is formed from a flexible material, and the lid part comprises a projection part configured to fit into the opening of the container when the lid part closes the opening so that when side walls of the container are pressed and bent, the projection part slips upward on an inner surface of the container and detaches from the container.
